# EUROPÄISCHE PATENTANMELDUNG

(11) **EP 0 869 109 A1**
(43) Veröffentlichungstag der Anmeldung: **07.10.1998**
(21) Anmeldenummer: 98105176.6
(22) Anmeldetag: 23.03.1998
(51) Int. Cl.: C07C 45/45

(54) **Verfahren zur Herstellung von 9-Anthracenaldehyden**

(30) Priorität: 04.04.1997 DE 19713912
(71) Anmelder: BAYER AG, 51368 Leverkusen (DE)
(72) Erfinder: Eichinger, Wolfram, Dr., 51061 Köln (DE)

(57) **Zusammenfassung**

9-Anthracenylaldehyde werden in besonders vorteilhafter Weise erhalten, indem man entsprechende Anthracenderivate mit Dimethylformamid in Gegenwart von Phosphorylchlorid umsetzt, das dabei erhältliche Reaktionsgemisch hydrolysiert und den gebildeten 9-Anthracenylaldehyd durch Versetzen mit einer Base ausfällt.

## Beschreibung

Die vorliegende Erfindung betrifft ein günstiges Verfahren zur Herstellung von 9-Anthracenylaldehyden. 9-Anthracenylaldehyde sind wertvolle Zwischenprodukte bei der Herstellung von Pflanzenschutzmitteln und Pharmazeutika.

Aus DRP 519 444 ist eine Herstellung von 9-Anthracenylaldehyd bekannt, bei der Anthracen mit N-Methylformanilid in Gegenwart von Phosphorylchlorid (= Phosphoroxychlorid = POCl₃) bei 80°C umgesetzt, anschließend in verdünnte Salzsäure eingetragen, nach kurzem Erhitzen das Produkt gefällt und aus Eisessig umkristallisiert wird. Dieses Verfahren wurde später durch die Verwendung von 1,2-Dichlorbenzol als Lösungsmittel verbessert (Org. Synth. Coll. Vol. III, S. 98 (1958)), wobei Ausbeuten von bis zu 84 % erzielt werden. Diese Arbeitsweise beinhaltet jedoch verschiedene Nachteile. So muß als Nebenprodukt anfallendes N-Methylanilin umweltgerecht entsorgt oder bei der Herstellung von N-Methylformanilid wiederverwendet und im Kreis geführt werden. Die Abtrennung von N-Methylanilin und gegebenenfalls von 1,2-Dichlorbenzol erfolgt durch Wasserdampfdestillation. Da auch 9-Anthracenaldehyd mit Wasserdampf flüchtig ist, geht ein Teil des Produktes verloren. Schließlich muß das zunächst erhaltene schwarze Öl in jedem Fall zur Reinigung umkristallisiert werden.

Bei Verwendung von Dimethylformamid an Stelle von N-Methylformamid konnte mit 1,2-Dichlorbenzol als Lösungsmittel eine Ausbeute von 62,5 % und bei Verwendung von Dimethylformamid auch als Lösungsmittel eine Ausbeute von 45 % erzielt werden (J. Am. Chem. Soc. 75, 989 (1953)), wobei auch hier auf die Umkristallisation des Produktes nicht verzichtet werden kann. Die Herstellung von 9-Anthracenaldehyd gelingt in nahezu quantitativer Ausbeute durch Reaktion von Anthracen mit einem Trifluormethansullonsäureanhydrid/Dimethylformamid-Komplex. Bei diesem Verfahren muß jedoch das anfallende Natriumtrifluormethansulfonat aus ökonomischen und ökologischen Gründen zurückgewonnen und wieder in das Anhydrid überführt werden.

Es wurde nun ein Verfahren zur Herstellung von 9-Anthracenylaldehyden der Formel (I) gefunden 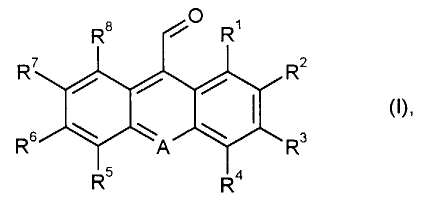 in der
- A: für C-R⁹ oder N und
- R¹ bis R⁹: unabhängig voneinander jeweils für Wasserstoff, Halogen, C₁-C₆-Alkoxy, Phenyl, Naphthyl, Anthracenyl, C₁-C₄-Alkyl oder C₃-C₇-Cycloalkyl stehen,
das dadurch gekennzeichnet ist, daß man ein Anthracenderivat der Formel (II) 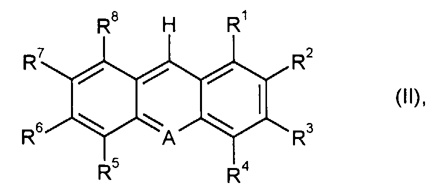 in der
- A und R¹ bis R⁹: die bei Formel (I) angegebene Bedeutung haben,
mit Dimethylformamid in Gegenwart von Phosphorylchlorid umsetzt, das dabei erhältliche Reaktionsgemisch hydrolysiert und den gebildeten 9-Anthracenylaldehyd durch Versetzen mit einer Base ausfällt.

In den Formeln (I) und (II) stehen R¹ bis R⁹ unabhängig voneinander bevorzugt für Wasserstoff, Fluor, Chlor, Brom, Methoxy, Ethoxy, Methyl, Ethyl, n-Propyl, i-Propyl, n-Butyl oder t-Butyl.

Weiterhin stehen vorzugsweise bis zu 4 der Reste R¹ bis R⁹ für einen der angegebenen Substituenten und die verbleibenden Reste für Wasserstoff. Besonders bevorzugt stehen bis zu 2 der Reste R¹ bis R⁹ für einen der angegebenen Substituenten und die verbleibenden Reste für Wasserstoff. Ganz besonders bevorzugt setzt man unsubstituiertes Anthracen ein.

Das erfindungsgemäße Verfahren kann beispielsweise bei 50 bis 150°C, vorzugsweise 80 bis 120°C, durchgeführt werden. Man kann bei Normaldruck, erhöhtem Druck oder erniedrigtem Druck arbeiten. Bevorzugt ist Normaldruck.

Pro Mol Anthracenderivat der Formel (I) kann man beispielsweise 1 bis 5 Mol, vorzugsweise 1 bis 2,5 Mol Phosphorylchlorid und 1 bis 5 Mol, vorzugsweise 1 bis 2,5 Mol Dimethylformamid einsetzen. Pro Mol Phosphorylchlorid kann man beispielsweise 0,2 bis 1,5 Mol, vorzugsweise 0,5 bis 1 Mol Dimethylformamid einsetzen.

Man kann das erfindungsgemäße Verfahren beispielsweise so durchführen, daß man das Anthracenderivat der Formel (I) und das Phosphorylchlorid vorlegt und Dimethylformamid bei der gewünschten Reaktionstemperatur zudosiert, beispielsweise im Verlauf von 0,5 bis 48 Stunden. Zudosierzeiten von 2 bis 18 Stunden sind bevorzugt. Man kann die Reaktionspartner auch in einer anderen Reihenfolge zusammengeben.

Nach Beendigung der Zugabe des letzten Reaktanden ist es zweckmäßig, noch einige Zeit innerhalb des Temperaturbereichs 50 bis 150°C nachzurühren. Die Nachrührzeit kann z.B. 1 bis 30 Stunden betragen.

Die Hydrolyse kann man im einfachsten Fall so durchführen, daß man das Reaktionsgemisch auf Wasser austrägt. Dabei findet die Entwicklung von Chlorwasserstoff statt, und es bildet sich eine dunkelrotbraune Lösung. Schließlich fällt man den gebildeten 9-Anthracenylaldehyd durch Zugabe einer Base aus. Beispielsweise kann man hierfür Natriumhydroxid oder wäßrige Natronlauge nehmen.

Es ist günstig, wenn die Temperatur des Wassers, mit dem man die Hydrolyse durchführt, zu Beginn beispielsweise auf 30 bis 70°C einstellt und während der Hydrolyse bei 35 bis 90°C hält. Die Zugabe einer Base kann beispielsweise so erfolgen, daß danach ein pH-Wert von 0,5 bis 7, vorzugsweise 1 bis 5,5, vorliegt. Während der Basenzugabe ist es vorteilhaft, die Temperatur im Bereich 10 bis 30°C zu halten und anschließend auf 20 bis 90°C, insbesondere 30 bis 80°C, zu erhöhen und noch einige Zeit, z.B. 0,5 bis 5 Stunden innerhalb dieses Temperaturbereichs nachzurühren.

Der hergestellte 9-Anthracenylaldehyd der Formel (I) liegt dann als Niederschlag vor und kann durch mechanische Abtrennung, beispielsweise durch Filtration, isoliert werden.

Wenn man den so erhaltenen 9-Anthracenylaldehyd weiter reinigen möchte, so kann man ihn z.B. mit Wasser anrühren, die sich bildende Suspension beispielsweise für 0,5 bis 5 Stunden bei 20 bis 80°C rühren, erneut abfiltrieren und mit Wasser nachwaschen. Man kann so 9-Anthracenylaldehyde in Ausbeuten von beispielsweise über 97 % und Reinheiten von über 98 % erhalten.

Im Vergleich zum Stand der Technik liefert das erfindungsgemäße Verfahren 9-Anthracenylaldehyde der Formel (I) auf einfacherem und wirtschaftlicherem Weg und in besseren Ausbeuten und Reinheiten. Der Anfall von Nebenprodukten, organisch belasteten Abwässern und Salzen (Natriumchlorid, Phosphate, Dimethylammoniumsalze), wird auf ein Minimum reduziert, langwierige Aufarbeitungsschritte, z.B. Wasserdampfdestillation, Umkristallisation, Rückführung von Reaktanden und Rückgewinnung von Lösungsmitteln, entfallen.

### Beispiele

### Beispiel 1

In einem Rührgefäß mit Rückflußkühler wurden 520 g Phosphorylchlorid vorgelegt, 300 g Anthracen zugegeben und bei 85 bis 90°C innerhalb von 7 Stunden unter Rühren 165 g Dimethylformamid zugetropft. Anschließend wurde noch 19 Stunden bei 85 bis 90°C nachgerührt. Nach dieser Zeit betrug der Umsatz 99,8 %. Die erhaltene heiße dunkelrote bis schwarze Reaktionsmischung wurde in eine auf 50°C geheizte und gerührte Vorlage von 2 l Wasser so eingetragen, daß die Temperatur bei 50 bis 60°C gehalten wurde. Dabei trat eine heftige Entwicklung von Chlorwasserstoffgas auf, das über einen Rückflußkühler abgeführt wurde. Die erhaltene Reaktionsmischung wurde auf Raumtemperatur abgekühlt. Durch portionsweise Zugabe von festem Natriumhydroxid (ca. 240 g), wobei die Temperatur bei 30°C gehalten wurde, wurde ein pH-Wert von 3,5 eingestellt und dann 1,5 Stunden bei 60°C nachgerührt. Dabei fiel 9-Anthracenaldehyd, zusammen mit wenig unumgesetztem Anthracen, quantitativ aus. Der Niederschlag wurde abfiltriert und weitestgehend von der Mutterlauge befreit. Der zurückbleibende Filterkuchen wurde erneut in 2 l Wasser suspendiert, 1,5 Stunden bei 60°C gerührt, abfiltriert und der erhaltene Filterkuchen mit 500 ml Wasser gewaschen.

Nach einer Trocknung bei 60°C wurden 340 g 9-Anthracenaldehyd erhalten, was einer Ausbeute von 98 % entspricht. Der Schmelzpunkt des Produktes betrug 104°C, der Gehalt an 9-Anthracenaldehyd 99,7 % (bestimmt nach GC) und der Gehalt an Anthracen 0,3 % (bestimmt nach GC). Mittels Elementaranalyse wurden noch folgende Werte ermittelt:
- Gehalt an Phosphor:: <0,05 %
- Gehalt an Stickstoff:: <0,01 %
- Gehalt an Natrium:: <0,03 %
- Gehalt an Chlor:: <0,05 %.

## Patentansprüche

1. Verfahren zur Herstellung von 9-Anthracenylaldehyden der Formel (I) 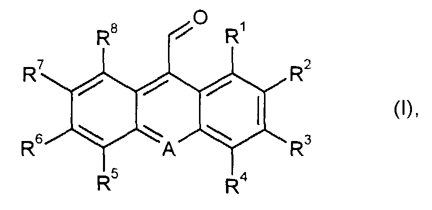 in der
A für C-R⁹ oder N und
R¹ bis R⁹ unabhängig voneinander jeweils für Wasserstoff, Halogen, C₁-C₆-Alkoxy, Phenyl, Naphthyl, Anthracenyl, C₁-C₄-Alkyl oder C₃-C₇-Cycloalkyl stehen,
dadurch gekennzeichnet, daß man ein Anthracenderivat der Formel (II) 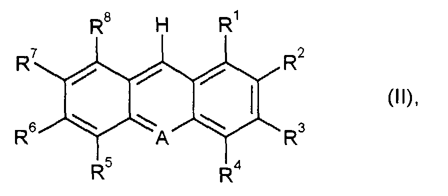 in der
A und R¹ bis R⁹ die bei Formel (I) angegebene Bedeutung haben,
mit Dimethylformamid in Gegenwart von Phosphorylchlorid umsetzt, das dabei erhältliche Reaktionsgemisch hydrolysiert und den gebildeten Anthracenylaldehyd durch Versetzen mit einer Base ausfällt.

2. Verfahren nach Anspruch 1, dadurch gekennzeichnet, daß in den Formeln (I) und (II) bis zu vier der Reste R¹ bis R⁹ für einen der angegebenen Substituenten und die verbleibenden Reste für Wasserstoff stehen.

3. Verfahren nach Ansprüchen 1 und 2, dadurch gekennzeichnet, daß in den Formeln (I) und (II) R¹ und R² unabhängig voneinander für Wasserstoff Fluor, Chlor, Brom, Methoxy, Ethoxy, Methyl, Ethyl, n-Propyl, i-Propyl, n-Butyl oder t-Butyl stehen.

4. Verfahren nach Ansprüchen 1 bis 3, dadurch gekennzeichnet, daß man die Umsetzung bei 50 bis 150°C und Normaldruck durchführt.

5. Verfahren nach Ansprüchen 1 bis 4, dadurch gekennzeichnet, daß man pro Mol Anthracenderivat der Formel (I) 1 bis 5 Mol Phosphorylchlorid und 1 bis 5 Mol Dimethylformamid einsetzt, wobei man pro Mol Phosphorylchlorid 0,2 bis 1,5 Mol Dimethylformamid verwendet.

6. Verfahren nach Ansprüchen 1 bis 5, dadurch gekennzeichnet, daß man das Anthracenderivat der Formel (I) und das Phosphoroxychlorid vorlegt und Dimethylformamid bei der gewünschten Reaktionstemperatur im Verlaufe von 0,5 bis 48 Stunden zudosiert.

7. Verfahren nach Ansprüchen 1 bis 6, dadurch gekennzeichnet, daß man die Hydrolyse durchführt, indem man das Reaktionsgemisch auf Wasser austrägt und dabei die Temperatur im Bereich 35 bis 90°C hält.

8. Verfahren nach Ansprüchen 1 bis 7, dadurch gekennzeichnet, daß man als Base Natriumhydroxid oder wäßrige Natronlauge bei 10 bis 30°C so zuführt, daß danach ein pH-Wert von 0,5 bis 7 vorliegt.

9. Verfahren nach Ansprüchen 1 bis 8, dadurch gekennzeichnet, daß man nach Beendigung der Basenzugabe noch 0,5 bis 5 Stunden bei 20 bis 90°C nachrührt.

10. Verfahren nach Ansprüchen 1 bis 9, dadurch gekennzeichnet, daß man den nach der Hydrolyse und Basenbehandlung vorliegenden Niederschlag abfiltriert, den Filterkuchen nochmal mit Wasser bei 20 bis 80°C 0,5 bis 5 Stunden verrührt, erneut abfiltriert und nachwäscht.
